# EUROPEAN PATENT APPLICATION

(11) **EP 3 045 163 A1**
(43) Date of publication of application: **20.07.2016**
(21) Application number: 16156221.0
(22) Date of filing: 19.04.2012
(51) Int. Cl.: A61K 9/00, A61K 9/08, A61K 47/10, A61K 47/32, A61K 47/34, A61K 47/38

(54) **OPHTHALMIC COMPOSITION WITH A VISCOSITY ENHANCEMENT SYSTEM HAVING TWO DIFFERENT VISCOSITY ENHANCING AGENTS**

(30) Priority: 22.04.2011 US 201161478084 P
(62) Divisional of application: 12718822.5
(71) Applicant: Alcon Research, Ltd., Fort Worth, Texas 76134-2099 (US)
(72) Inventor: CHOWHAN, Masood A., Arlington, TX Texas 76016 (US); GHOSH, Malay, Fort Worth, TX Texas 76019 (US)
(74) Representative: Teipel, Stephan

(57) **Abstract**

An ophthalmic composition is disclosed having a viscosity enhancement system comprised of two different viscosity enhancing agents. The aqueous composition contains a first viscosity enhancing agent that provides enhanced viscosity upon dispensing of the composition to the eye and a second viscosity agent that increases viscosity (e.g., gels or partially gels) after dispensing of the composition to the eye to provide extended viscosity enhancement of the composition.

## Description

### Cross-Reference to Related Application

The present application claims priority based on U.S. Provisional Patent Application Serial No. 61/478,084 filed April 22, 2011.

### Technical Field of the Invention

The present invention relates to an ophthalmic composition with a viscosity enhancement system comprised of two different viscosity enhancing agents. More particularly, the present invention relates to an ophthalmic aqueous composition containing a first viscosity enhancing agent that provides enhanced viscosity upon dispensing of the composition to the eye and a second viscosity agent that increases viscosity (e.g., gels or partially gels) after dispensing of the composition to the eye to provide extended viscosity enhancement of the composition.

### Background of the Invention

It is known that ophthalmic compositions, which are topically delivered to the ocular surface of an eye, can provide significant benefits if those composition have enhanced viscosity. For example, it has been found that an enhanced viscosity aqueous ophthalmic composition can often provide enhanced penetration of a therapeutic agent into the eye relative to a similar aqueous composition having a lower viscosity. As another example, an enhanced viscosity aqueous ophthalmic composition can provide greater relief of dry eye symptoms relative to a similar aqueous ophthalmic composition having a lower viscosity.

Based upon this knowledge, the ophthalmic industry has expended substantial effort and substantial resources in developing ophthalmic compositions with enhanced viscosity. As a result, numerous viscosity enhancing agents, mostly polymeric agents, have been tested for their ability to enhance the viscosity of ophthalmic solutions and several of these agents have been widely used in ophthalmic solutions. Examples of polymers that have been tested or used include, without limitation, carboxyvinyl polymer, cellulosic polymers (e.g., carboxymethyl cellulose, hydroxypropylmethyl cellulose, hydroxyethyl cellulose or the like), polysaccharides (e.g., xanthan gum), polyvinyl pyrrolidone, polyvinyl alcohol and many others.

In addition to use of viscosity enhancers in general, there has also been development of viscosity enhancement systems that operate in conjunction with tear fluid and/or other chemical entities to provide desired viscosity enhancement to topical aqueous ophthalmic compositions. As one example, U.S. Patent No. 7,169,767, which is incorporated herein by reference in its entirety for all purposes, discloses a galactomannan-borate system that gels or partially gels upon administration to the eye. While this system is particularly desirable in many respects, the system takes time to gel upon the eye such that the enhanced viscosity effect is not instantaneous.

While enhanced viscosity upon administration to the eye has been found to improve ophthalmic compositions, it is often also desirable for that viscosity to remain enhanced for an extended period of time after administration of the aqueous ophthalmic composition to the surface of the eye. This extended time viscosity enhancement is particularly desirable for enhancing penetration of a therapeutic agent into the eye. In an effort to achieve these extended time periods of enhanced viscosity, the ophthalmic industry has focus upon discovering and developing viscosity enhancing agents, particularly polymers, that have viscoelastic properties and mucoadhesive properties that aid in maintaining a viscosity enhancing agent upon the surface of the eye for a greater period of time. While significant achievements have been made in this regard, these polymers, even with their enhanced properties, often disperse and dissolve away too quickly in ocular tear fluid. Moreover, some of these polymers may be undesirable on the surface of the eye because they can cause impairment of vision and other undesirable effects.

In view of the above, it will be understood that the ophthalmic industry continues to pursue breakthroughs in viscosity enhancement of topical aqueous ophthalmic compositions for both dry eye and for the delivery of therapeutic agents. As such, the present invention provides an ophthalmic aqueous composition containing a first viscosity enhancing agent that provides enhanced viscosity upon dispensing of the composition to the eye and a second viscosity agent that increases viscosity (e.g., gels or partially gels) after dispensing of the composition to the eye to provide an improved extended viscosity enhancement of the composition.

### Summary of the Invention

The present invention is directed to a topical ophthalmic multi-dose aqueous composition comprising a viscosity enhancing system and water. The system includes a dissipation viscosity enhancing agent and a thermally sensitive viscosity agent. The dissipation viscosity enhancing agent exhibits enhanced viscosity upon administration of the composition to an ocular surface of a human eye but then dissipates and gradually loses viscosity thereafter. The thermally sensitive viscosity enhancing agent exhibits a lower viscosity upon administration of the composition to the ocular surface of the human eye but then exhibits enhanced viscosity after administration to the ocular surface of the eye. The composition is particularly desirable for delivery of a therapeutic agent to the eye. Examples of suitable thermally sensitive agent include ethyl hydroxyethyl cellulose, methyl cellulose, polaxamer (e.g., nonioinic triblock copolymers composed of a central hydrophobic chain of polyoxypropylene (poly(propylene oxide)) flanked by two hydrophilic chains of polyoxyethylene (poly(ethylene oxide)) (e.g., PLURONICS® commercially available from BASF), block copolymer formed of poly-(lactide-co-glycolide) and polyethylene glycol (e.g., an ABA tri-block copolymer), acrylamide such as poly (N-isopropylacrylamide) and/or poly(N,N-dimethylacrylamide), tetra-functional block copolymers based on ethylene oxide and propylene oxide (e.g., TETRONICS® commercially available from BASF), any combination thereof or the like. Examples of suitable dissipation viscosity enhancing agent include carboxyvinyl polymer, HPMC, HEC, CMC, polyvinyl alcohol, polyvinyl pyrrolidone (PVP) or any combination thereof. Both the dissipation viscosity enhancing agent and the ion sensitive viscosity enhancing agent can be polymers.

The present invention is also directed to a method of topically administering an ophthalmic composition to an eye of a mammal. The composition can be as described above or otherwise herein. The mammal will typically be a human being. In a preferred embodiment, the composition is administered by releasing an eyedrop of the composition from an eyedropper to the eye.

The present invention has the following embodiments:
1. A topical ophthalmic multi-dose aqueous composition comprising:
   a viscosity enhancing system comprised of:
      i) dissipation viscosity enhancing agent that exhibits enhanced viscosity upon administration of the composition to an ocular surface of a human eye but then dissipates and gradually loses viscosity thereafter; and
      ii) thermally sensitive phase transition viscosity enhancing agent that exhibits a lower viscosity upon administration of the composition to the ocular surface of the human eye but then exhibits enhanced viscosity after administration to the ocular surface of the eye; and
         water.
2. An ophthalmic composition as in embodiment 1 further comprising a therapeutic agent.
3. An ophthalmic composition as in embodiment 1 or 2 wherein the thermally sensitive polymer is selected from the group consisting of ethyl hydroxyethyl cellulose, methyl cellulose, polaxamer (e.g., nonioinic triblock copolymers composed of a central hydrophobic chain of polyoxypropylene (poly(propylene oxide)) flanked by two hydrophilic chains of polyoxyethylene (poly(ethylene oxide)) (e.g., PLURONICS® commercially available from BASF), block copolymer formed of poly-(lactide-co-glycolide) and polyethylene glycol (e.g., an ABA tri-block copolymer), acrylamide such as poly (N-isopropylacrylamide) and/or poly(N,N-dimethylacrylamide), tetra-functional block copolymers based on ethylene oxide and propylene oxide (e.g., TETRONICS® commercially available from BASF) or any combination thereof.
4. An ophthalmic composition as in embodiment 1, 2 or 3 wherein the dissipation polymer is selected from the group consisting of carboxyvinyl polymer, HPMC, HEC, PVP, CMC, polyvinyl alcohol or any combination thereof.
5. An ophthalmic composition as in any of the preceding embodiments wherein the dissipation viscosity enhancing agent provides the composition with an additional viscosity that is at least 10 cp but is no greater than 100 cp.
6. An ophthalmic composition as in any of the preceding embodiments wherein the concentration of the dissipation viscosity enhancing agent in the composition is at least about 0.10 w/v% but no greater than about 2.5 w/v%.
7. An ophthalmic composition as in any of the preceding embodiments wherein the concentration of the thermally sensitive viscosity agent in the composition is at least about 0.80 w/v%, but no greater than 10 w/v%.
8. An ophthalmic composition as in any of the preceding embodiments wherein the thermally sensitive viscosity agent substantially increases viscosity and preferably gels or at least partially gels at an elevated temperature that is between room temperature (i.e., 25 °C) and human body temperature (i.e., 37 °C), more typically between 29 °C and 35 °C and even more typically between 32 °C and 34 °C.
9. A topical ophthalmic multi-dose aqueous composition comprising:
   a therapeutically effective amount of therapeutic agent;
   a viscosity enhancing system comprised of:
      i) dissipation viscosity enhancing agent that exhibits enhanced viscosity upon administration of the composition to an ocular surface of a human eye but then dissipates and gradually loses viscosity thereafter, the dissipation viscosity enhancing agent being polymeric; and
      ii) thermally sensitive phase transition viscosity enhancing agent that exhibits a lower viscosity upon administration of the composition to the ocular surface of the human eye but then exhibits enhanced viscosity after administration to the ocular surface of the eye, the dissipation viscosity enhancing agent being polymeric; and
         water.
10. An ophthalmic composition as in embodiment 9 wherein the thermally sensitive polymer is selected from the group consisting of ethyl hydroxyethyl cellulose, methyl cellulose, polaxamer (e.g., nonioinic triblock copolymers composed of a central hydrophobic chain of polyoxypropylene (poly(propylene oxide)) flanked by two hydrophilic chains of polyoxyethylene (poly(ethylene oxide)) (e.g., PLURONICS® commercially available from BASF), block copolymer formed of poly-(lactide-co-glycolide) and polyethylene glycol (e.g., an ABA tri-block copolymer), acrylamide such as poly (N-isopropylacrylamide) and/or poly(N,N-dimethylacrylamide), tetra-functional block copolymers based on ethylene oxide and propylene oxide (e.g., TETRONICS® commercially available from BASF) or any combination thereof.
11. An ophthalmic composition as in embodiment 9 or 10 wherein the dissipation polymer is selected from the group consisting of carboxyvinyl polymer, HPMC, HEC, CMC or any combination thereof.
12. An ophthalmic composition as in any of embodiments 9-11 wherein the dissipation viscosity enhancing agent provides the composition with an additional viscosity that is at least 10 cp but is no greater than 100 cp.
13. An ophthalmic composition as in any of embodiments 9-12 wherein the concentration of the dissipation viscosity enhancing agent in the composition is at least about 0.10 w/v% but no greater than about 2.5 w/v%.
14. An ophthalmic composition as in any of embodiments 9-13 wherein the concentration of the thermally sensitive viscosity agent in the composition is at least about 0.80 w/v%, but no greater than 10 w/v%.
15. An ophthalmic composition as in any of embodiments 9-14 wherein the thermally sensitive viscosity agent substantially increases viscosity and preferably gels or at least partially gels at an elevated temperature that is between room temperature (i.e., 25 °C) and human body temperature (i.e., 37 °C), more typically between 29 °C and 35 °C and even more typically between 32 °C and 34 °C.
16. An ophthalmic composition as in any of the preceding embodiments wherein the therapeutic agent is selected from the group consisting of anti-glaucoma agents, anti-angiogenesis agents; anti-infective agents; anti-inflammatory agents; growth factors; immunosuppressant agents; and anti-allergic agents.
17. A method of administering an ophthalmic composition as in any of the preceding embodiments, comprising:
   topically administering the ophthalmic composition to an eye of a mammal.
18. A method as in embodiment 17 wherein the mammal is a human being.
19. A method as in embodiment 17 or 18 wherein the step of administering includes releasing an eyedrop of the composition from an eyedropper to the eye.

### Detailed Description of the Invention

The present invention is predicated upon the provision of an aqueous ophthalmic composition with a viscosity enhancement system comprised of two different viscosity enhancing agents. The viscosity enhancement system includes a first viscosity enhancing agent (typically referred to herein as a dissipation viscosity enhancing agent) that provides enhanced viscosity upon dispensing of the composition to the eye. The viscosity enhancing system also includes a second viscosity agent (typically referred to as thermally sensitive viscosity enhancing agent) that increases viscosity (e.g., gels or partially gels) after dispensing of the composition to the eye to provide extended viscosity enhancement of the composition. The ophthalmic composition is preferably an ophthalmic aqueous composition such as a multi-dose ophthalmic aqueous solution. The ophthalmic composition is particularly desirable for delivery of therapeutic agents to the eye and for use in alleviating dry eye symptoms. A two viscosity agent system that will exhibit characteristics similar to the viscosity system of the present invention is described in U.S. Patent Application Serial No. 12/957,864, titled "Carboxyvinyl Polymer-Containing Nanoparticles Suspensions", filed December 1, 2010 and incorporated herein in its entirety for all purposes.

Unless otherwise stated, concentrations of ingredients in the compositions of the present invention are provided in weight/volume percentage (w/v%).

Unless otherwise stated, viscosities of the compositions discussed herein are determined with a Brookfield viscometer using cone and plate configuration at 3-60 rpm and a temperature of 25 °C.

The first viscosity enhancing agent of the present invention provides enhanced viscosity to the ophthalmic composition both prior to dispensing the composition, upon dispensing of the composition and for a period of time thereafter. After dispensing, the ability of the first viscosity enhancing agent to maintain enhanced viscosity dissipates and the first viscosity enhancing agent is therefore also referred to herein as the dissipation viscosity enhancing agent. While it is contemplated that the first viscosity enhancing agent may exhibit some degree of viscosity enhancement after dispensing of the composition to the eye, it is to be understood that the dissipation viscosity enhancing agent will disperse within the tear fluid of the eye after dispensing and its ability to provide viscosity enhancement will dissipate during a period of time in which the second or thermally sensitive viscosity enhancing agent is gaining ability to provide viscosity enhancement of the composition. This is described in further detail below.

The dissipation viscosity enhancing agent is typically a polymer, although not necessarily required unless otherwise specifically stated. Examples of suitable viscosity enhancing agent includes, without limitation, carboxyvinyl polymer (e.g., Carbopol 934P or 974P, commercially available from The Lubrizol Corporation, headquartered in Wickliffe, Ohio), hydroxyethyl cellulose (HEC), hydroxypropylmethyl cellulose (HPMC), carboxymethylcellulose (CMC), polyvinyl alcohol, PVP, any combination thereof or the like.

The concentration of the dissipation viscosity enhancing agent within the ophthalmic composition can vary depending upon the specific type of agent[s] used and the desired viscosity of the composition. Typically, however, the concentration of the dissipation viscosity enhancing agent within the ophthalmic composition is at least about 0.05 w/v%, more typically at least about 0.10 w/v%" more typically at least about 0.80 w/v%" possibly at least 1.4 w/v%, and even possibly at least 1.9 w/v% and typically no greater than about 5.0 w/v%, more typically no greater than 3.0 w/v%, more typically no greater than 2.5 w/v%, possibly no greater than 1.9 w/v% and even possibly no greater than 0.8 w/v%.

The dissipation viscosity enhancing agent will typically be the primary provider of viscosity within the composition prior to dispensing of the composition to the eye (e.g., when the composition is in a dispensing container such as an eyedropper). The dissipation viscosity enhancing agent will typically provide the composition with an additional viscosity that is at least 5 centipoise (cp), more typically at least 10 cp, even more typically at least 20 cp, possibly at least 40 cp and even possibly at least 60 cp greater than a viscosity of the composition without the dissipation viscosity enhancing agent. That same additional viscosity, however, is typically no greater than about 500 cp more typically no greater than 100 cp, more typically no greater than 75 cp, possibly no greater than 30 cp and even possibly no greater than 15 cp greater than the viscosity of the composition without the dissipation viscosity enhancing agent. As used herein, the viscosity of the composition without the dissipation viscosity enhancing agent is measured by forming a composition that is identical to the composition in question with the exception that the dissipation viscosity enhancing agent has been replaced with water.

The second viscosity enhancing agent of the present invention provides enhanced viscosity to the ophthalmic composition after dispensing of the composition and for a period of time thereafter. After dispensing, the ability of the second viscosity enhancing agent to maintain enhanced viscosity increases. Typically, the second viscosity agent increases viscosity (e.g., gels or partially gels) after administration of the composition to the surface of the eye. Preferably, the second viscosity agent is sensitive to changes in temperature such that, after dispensing to the eye, the composition mixes with tear fluid and is heated toward body temperature causing the second viscosity enhancing agent to increase its viscosity and the viscosity of the composition, for example, through gelling or partial gelling thereof. Thus, the second viscosity agent is referred to herein as the thermally sensitive viscosity enhancing agent. It shall be understood that the thermally sensitive viscosity enhancing agent enhances the viscosity of portions of the composition after administration to the eye to the extent that those portions of the composition have not been already dispersed away by the tear fluid. Hence, the statement that the thermally sensitive viscosity agent increases the viscosity of the composition after administration to the eye only requires that a portion of the composition have its viscosity enhanced.

The enhancement in viscosity provided by the thermally sensitive viscosity enhancing agent occurs, at least in part, during a time period in which the dissipation viscosity enhancing agent is losing its ability to provide enhanced viscosity. In this manner, the composition of the present invention provides a consistently enhanced viscosity over a period of time that begins immediately upon dispensing of the composition by virtue of the dissipation viscosity enhancing agent and continues for a significant period of time thereafter (i.e., after significant dispersion of the dissipation viscosity agent) by virtue of the thermally sensitive viscosity enhancing agent.

The thermally sensitive viscosity enhancing agent is typically a polymer, although not necessarily required unless otherwise specifically stated. The thermally sensitive viscosity agent is typically comprised of one or more agents that increase viscosity by hydrolyzing and/or gelling or partially gelling upon an increase in temperature of the thermally sensitive viscosity agent. Thus, the thermally sensitive viscosity agent substantially increases viscosity and preferably gels or at least partially gels at an elevated temperature that is between room temperature (i.e., 25 °C) and human body temperature (i.e., 37 °C), more typically between 29 °C and 35 °C and even more typically between 32 °C and 34 °C. As used herein, the phrase "at least partially gelling" and its derivations include any further gelling of any already gelled solution or ingredient. Also, as used herein, the term "gelling" and its derivations include any slight or fuller gelling of a non-gelled solution or ingredient. Further, as used herein, "substantially increases viscosity" means an increase of viscosity of at least 20% (e.g., 100 cp to 120 cp), more preferably at least 40% for just the thermally sensitive viscosity agent.

Preferably, the thermally sensitive viscosity agent undergoes physical and/or chemical change (e.g., cross-linking, domain formation, complexation, polymerization or the like) at the aforementioned elevated temperature to increase viscosity and preferably gel or partially gel. Examples of suitable thermally sensitive viscosity enhancing agent includes, without limitation, ethyl hydroxyethyl cellulose, methyl cellulose, polaxamer (e.g., nonioinic triblock copolymers composed of a central hydrophobic chain of polyoxypropylene (poly(propylene oxide)) flanked by two hydrophilic chains of polyoxyethylene (poly(ethylene oxide)) (e.g., PLURONICS® commercially available from BASF), block copolymer formed of poly-(lactide-co-glycolide) and polyethylene glycol (e.g., an ABA tri-block copolymer), acrylamide such as poly (N-isopropylacrylamide) and/or poly(N,N-dimethylacrylamide), tetra-functional block copolymers based on ethylene oxide and propylene oxide (e.g., TETRONICS® commercially available from BASF), any combination thereof or the like. One preferred block polymer is sold under the tradename REGEL® and is commercially available from Protherics, London, England.

The concentration of the thermally sensitive viscosity enhancing agent within the ophthalmic composition can vary depending upon the specific type of agent[s] used and the desired viscosity of the composition. Typically, however, the concentration of the thermally sensitive viscosity enhancing agent within the ophthalmic composition is at least about 0.05 w/v%, more typically at least about 0.10 w/v%" more typically at least about 0.80 w/v%" possibly at least 1.5 w/v%, and even possibly at least 5 w/v% and typically no greater than about 22 w/v%, more typically no greater than 15 w/v%, more typically no greater than 10 w/v%, possibly no greater than 5 w/v% and even possibly no greater than 2 w/v%.

The thermally sensitive viscosity enhancing agent can provide viscosity within the composition prior to dispensing of the composition to the eye (e.g., when the composition is in a dispensing container such as an eyedropper). However, as discussed above, the dissipation viscosity enhancing agent will typically provide the composition with most of the viscosity prior to dispensing or administration of the composition to the eye. The thermally sensitive viscosity enhancing agent will enhance viscosity of the composition within the eye as the dissipation viscosity enhancing agent dissipates and loses its ability to provide viscosity enhancement.

It shall be understood that the thermally sensitive viscosity enhancing agent and the dissipation viscosity enhancing agent are always different from each other when considering a single composition of the present invention. It may be possible that a viscosity agent may be a dissipation viscosity agent in one composition of the present invention while that same viscosity agent may be a thermally sensitive viscosity agent in another composition of the present invention. The determining factor for whether the agent is one or the other is the characteristics of the agent prior to administration to the eye relative to its characteristics after administration.

In one preferred embodiment, the composition of the present invention will include an ophthalmically acceptable therapeutic agent. Non-limiting examples of potential ophthalmic therapeutic agents for the present invention include: anti-glaucoma agents, anti-angiogenesis agents; anti-infective agents; anti-inflammatory agents; growth factors; immunosuppressant agents; and anti-allergic agents. Anti-glaucoma agents include beta-blockers, such as betaxolol and levobetaxolol; carbonic anhydrase inhibitors, such as brinzolamide and dorzolamide; prostaglandins, such as travoprost, bimatoprost, and latanoprost; seretonergics; muscarinics; dopaminergic agonists. Anti-angiogenesis agents include anecortave acetate (RETAANE™, Alcon™ Laboratories, Inc. of Fort Worth, Tex.) and receptor tyrosine kinase inhibitors (RTKi). Anti-inflammatory agents include non-steroidal and steroidal anti-inflammatory agents, such as triamcinolone acetonide, dexamethasone, prednisolone, difluprednate, suprofen, diclofenac, ketorolac, nepafenac, rimexolone, and tetrahydrocortisol. Growth factors and growth factor promoters include EGF, PDGF or VEGF. Anti-allergic agents include olopatadine, emadastine and epinastine. Anti-infective agents include moxifloxacin, ciprofloxacin, gatifloxacin and ofloxacin. The ophthalmic drug may be present in the form of a pharmaceutically acceptable salt.

In another embodiment of the present invention, the composition is configured to provide relief of dry eye symptoms. In such an embodiment, the composition may be without any therapeutic agents designed to treat ocular disease other than dry eye. In a high preferred embodiment, ocular disease other than dry eye includes glaucoma or ocular hypertension, angiogenesis, infection, suppression of the immune system, inflammation unrelated to dry eye and allergy.

The composition of the present invention can include borate. As used herein, the term "borate" shall refer to boric acid, salts of boric acid, borate derivatives and other pharmaceutically acceptable borates, or combinations thereof. Most suitable are: boric acid, sodium borate, potassium borate, calcium borate, magnesium borate, manganese borate, and other such borate salts. Typically, when used, the borate is at least about 0.05 w/v %, more typically at least about 0.18 w/v % and even possibly at least about 0.27 w/v % of the ophthalmic composition and is typically less than about 1.0 w/v %, more typically less than about 0.75 w/v % and still more typically less than about 0.4 w/v %, and even possibly less than about 0.35 w/v % of the ophthalmic composition.

The composition of the present invention can also include polyol. As used herein, the term "polyol" includes any compound having at least one hydroxyl group on each of two adjacent carbon atoms that are not in *trans* configuration relative to each other. The polyol can be linear or cyclic, substituted or unsubstituted, or mixtures thereof, so long as the resultant complex is water soluble and pharmaceutically acceptable. Examples of such compounds include: sugars, sugar alcohols, sugar acids and uronic acids. Preferred polyols are sugars, sugar alcohols and sugar acids, including, but not limited to: mannitol, glycerin, xylitol, sorbitol and propylene glycol. It is contemplated that the polyol may be comprised of two or more different polyols.

When both borate and polyol are present in the compositions borate typically interacts with polyol, such as glycerol, propylene glycol, sorbitol and mannitol, or any combination thereof to form borate polyol complexes. The type and ratio of such complexes depends on the number of OH groups of a polyol on adjacent carbon atoms that are not in trans configuration relative to each other. It shall be understood that weight/volume percentages of the ingredients polyol and borate include those amounts whether as part of a complex or not. Advantageously, the borate and polyol can act as buffers and/or tonicity agents and can also aid in enhancing preservation efficacy of the composition.

The composition of the present invention can also include additional or alternative suitable buffer systems or ingredients including, but not limited to, tris, acetate or the like provided the buffer does not interfere with the thermally sensitive polymer.

The composition of the present invention typically includes a preservative. Potential preservatives include, without limitation, hydrogen peroxide, benzalkonium chloride (BAK), polymeric quaternary ammonium compound (PQAM), biguanides, chlorohexidine, sorbic acid or others. Of these, benzalkonium chloride and polymeric quaternary ammonium compound such as polyquaternium-1 have proven quite desirable.

The polymeric quaternary ammonium compounds useful in the compositions of the present invention are those which have an antimicrobial effect and which are ophthalmically acceptable. Preferred compounds of this type are described in U.S. Pat. Nos. 3,931,319; 4,027,020; 4,407,791; 4,525,346; 4,836,986; 5,037,647 and 5,300,287; and PCT application WO 91/09523 (Dziabo et al.). The most preferred polymeric ammonium compound is polyquaternium-1, otherwise known as POLYQUAD® or ONAMER_M® with a number average molecular weight between 2,000 to 30,000. Preferably, the number average molecular weight is between 3,000 to 14,000.

When used, the polymeric quaternary ammonium compound is generally used in the composition of the present invention in an amount that is greater than about 0.00001 w/v %, more typically greater than about 0.0003 w/v % and even more typically greater than about 0.0007 w/v % of the ophthalmic composition. Moreover, the polymeric quaternary ammonium compound is generally used in the composition of the present invention in an amount that is less than about 0.01 w/v %, more typically less than about 0.003 w/v % and even more typically less than about 0.0015 w/v % of the ophthalmic composition.

BAK is generally used in the composition of the present invention in an amount that is greater than about 0.001 w/v %, more typically greater than about 0.003 w/v % and even more typically greater than about 0.007 w/v % of the ophthalmic composition. Moreover, BAK is generally used in the composition of the present invention in an amount that is less than about 0.1 w/v %, more typically less than about 0.03 w/v % and even more typically less than about 0.015 w/v % of the ophthalmic composition.

It is also contemplated that the composition of the present invention may benefit from the use of two different polyols, borate and a preservative (e.g., BAK or polymeric quaternary ammonium compound) to provide enhanced preservations efficacy. Examples of such systems are disclosed in U.S. Patent Publication Nos. 2009/0232763 and 2010/0324031, which are expressly incorporated herein in their entirety for all purposes.

It is contemplated that the composition of the present invention can include a variety of additional ingredients. Such ingredients include, without limitation, additional therapeutic agents, additional or alternative antimicrobial agents, suspension agents, surfactants, additional or alternative tonicity agents, additional or alternative buffering agents, anti-oxidants, additional or alternative viscosity-modifying agents, chelating agents any combinations thereof or the like.

The compositions of the present invention will generally be formulated as sterile aqueous solutions. The compositions of the present invention are also formulated so as to be compatible with the eye and/or other tissues to be treated with the compositions. The ophthalmic compositions intended for direct application to the eye will be formulated so as to have a pH and tonicity that are compatible with the eye. It is also contemplated that the compositions can be suspensions or other types of solutions.

The composition of the present invention will typically have a pH in the range of 4 to 9, preferably 5.5 to 8.5, and most preferably 5.5 to 8.0. Particularly desired pH ranges are 6.0 to 7.8 and more specifically 6.4 to 7.2. The compositions will have an osmolality of 200 to 400 or 450 milliosmoles per kilogram (mOsm/kg), more preferably 240 to 360 mOsm/kg.

In a preferred embodiment, the composition of the present invention is a multi-dose ophthalmic compositions that have sufficient antimicrobial activity to allow the compositions to satisfy the USP preservative efficacy requirements, as well as other preservative efficacy standards for aqueous pharmaceutical compositions.

The preservative efficacy standards for multi-dose ophthalmic solutions in the U.S. and other countries/regions are set forth in the following table:

**Preservative Efficacy Test ("PET") Criteria (Log Order Reduction of Microbial Inoculum Over Time**

| | Bacteria | Fungi |
|---|---|---|
| USP 27 | A reduction of 1 log (90%), by day 7; 3 logs (99.9%) by day 14; and no increase after day 14 | The compositions must demonstrate over the entire test period, which means no increases of 0.5 logs or greater, relative to the initial inoculum. |
| Japan | 3 logs by 14 days; and no increase from day 14 through day 28. | No increase from initial count at 14 and 28 days |
| Ph. Eur. A¹ | A reduction of 2 logs (99%) by 6 hours; 3 logs by 24 hours; and no recovery after 28 days | A reduction of 2 logs (99%) by 7 days, and no increase thereafter |
| Ph. Eur. B | A reduction of 1 log at 24 hours; 3 logs by day 7; and no increase thereafter | A reduction of 1 log (90%) by day 14, and no increase thereafter |
| FDA/ISO 14730 | A reduction of 3 logs from initial challenge at day 14; and a reduction of 3 logs from rechallenge | No increase higher than the initial value at day 14, and no increase higher than the day 14 rechallenge count through day 28. |

| | | |
|---|---|---|
| ¹There are two preservative efficacy standards in the European Pharmacopoeia "A" and "B". | | |

The standards identified above for the USP 27 are substantially identical to the requirements set forth in prior editions of the USP, particularly USP 24, USP 25 and USP 26.

Applicants specifically incorporate the entire contents of all cited references in this disclosure. Further, when an amount, concentration, or other value or parameter is given as either a range, preferred range, or a list of upper preferable values and lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range. It is not intended that the scope of the invention be limited to the specific values recited when defining a range.

Other embodiments of the present invention will be apparent to those skilled in the art from consideration of the present specification and practice of the present invention disclosed herein. It is intended that the present specification and examples be considered as exemplary only with a true scope and spirit of the invention being indicated by the following claims and equivalents thereof.

Table A below provides a listing of exemplary ingredients suitable for an exemplary preferred formulation of the ophthalmic composition of the present invention and a desired weight/volume percentage for those ingredients. It shall be understood that certain ingredients can be added or removed from the ingredients in Table A concentrations may be varied for the ingredients and pH values may be varied while remaining within the scope of the present invention.

**TABLE A**

| Ingredient | w/v percent |
|---|---|
| Ophthalmically Acceptable Therapeutic Agent | 0.7 |
| Dissipation Viscosity Enhancing Agent | 0.8 |
| Thermally Sensitive Viscosity Enhancing Agent | 0.2 or 0.8 |
| Buffer (e.g., Borate) | 0.3 |
| Polyol (e.g., mannitol or propylene glycol) | 0.6 |
| Sodium Chloride | 0.35 |
| Preservative | 0.01 for BAK or 0.001 PQAM |
| NaOH or tromethamine/ HCl | sufficient to achieve pH = 7.0 |
| purified water | Q.S. 100 % |

It is understood that the weight/volume percents in table A can be varied by ±10%, ± 20%, ±30%, ±90% of those weight/volume percents or more and that those variances can be specifically used to create ranges for the ingredients of the present invention. For example, an ingredient weight/volume percent of 10% with a variance of ±20% means that the ingredient can have a weight/volume percentage range of 8 to 12 w/v %.

## Claims

1. A topical ophthalmic multi-dose aqueous composition comprising:
a viscosity enhancing system comprised of:
i) dissipation viscosity enhancing agent that exhibits enhanced viscosity upon administration of the composition to an ocular surface of a human eye but then dissipates and gradually loses viscosity thereafter wherein the concentration of the dissipation viscosity enhancing agent in the composition is at least about 0.10 w/v%, but no greater than about 2.5 w/v%; and
ii) thermally sensitive phase transition viscosity enhancing agent that exhibits a lower viscosity upon administration of the composition to the ocular surface of the human eye but then exhibits enhanced viscosity after administration to the ocular surface of the eye wherein the concentration of the thermally sensitive viscosity agent in the composition is at least about 0.80 w/v%, but no greater than 10 w/v%; and
water;
wherein the composition has a pH in the range of 6.0 to 7.8 and an osmolality of 200 to 450 mOsm/kg.

2. An ophthalmic composition as in claim 1 further comprising a therapeutic agent.

3. An ophthalmic composition as in claim 1 or 2, wherein the dissipation viscosity enhancing agent is polymeric.

4. An ophthalmic composition as in claim 1, 2 or 3 wherein the thermally sensitive polymer is selected from the group consisting of ethyl hydroxyethyl cellulose, methyl cellulose, polaxamer such as nonioinic triblock copolymers composed of a central hydrophobic chain of polyoxypropylene (poly(propylene oxide)) flanked by two hydrophilic chains of polyoxyethylene (poly(ethylene oxide), block copolymer formed of poly-(lactide-co-glycolide) and polyethylene glycol such as an ABA tri-block copolymer, acrylamide such as poly (N-isopropylacrylamide) and/or poly(N,N-dimethylacrylamide), tetra-functional block copolymers based on ethylene oxide and propylene oxide or any combination thereof.

5. An ophthalmic composition as in any of the preceding claims wherein the dissipation polymer is selected from the group consisting of carboxyvinyl polymer, HPMC, HEC, PVP, CMC, polyvinyl alcohol or any combination thereof.

6. An ophthalmic composition as in any of the preceding claims wherein the dissipation viscosity enhancing agent provides the composition with an additional viscosity that is at least 0.01 m-1 • kg • s⁻¹ (10 cp) but is no greater than 0.1 m⁻¹ • kg • s⁻¹ (100 cp).

7. An ophthalmic composition as in any of the preceding claims wherein the thermally sensitive viscosity agent substantially increases viscosity and preferably gels or at least partially gels at an elevated temperature that is between room temperature (i.e., 25 °C) and human body temperature (i.e., 37 °C), more typically between 29 °C and 35 °C and even more typically between 32 °C and 34 °C.

8. An ophthalmic composition as in any of claims 2-7 wherein the therapeutic agent is selected from the group consisting of anti-glaucoma agents, anti-angiogenesis agents; anti-infective agents; anti-inflammatory agents; growth factors; immunosuppressant agents; and anti-allergic agents.

9. An ophthalmic composition as in any of the preceding claims for use in a method of administrating, comprising:
topically administering the ophthalmic composition to an eye of a mammal.

10. An ophthalmic composition for use according to claim 9 wherein the mammal is a human being.

11. An opthalmic composition for use according to claim 9 or 10 wherein the step of administering includes releasing an eyedrop of the composition from an eyedropper to the eye.
